# FASCICULE DE BREVET EUROPEEN

(11) **EP 3 572 029 B1**
(45) Date de publication et mention de la délivrance du brevet: **21.09.2022**
(21) Numéro de dépôt: 18305627.4
(22) Date de dépôt: 22.05.2018
(51) Int. Cl.: A61C 7/00, A61C 9/00, G06T 7/00

(54) **DISPOSITIF D'ANALYSE D'UNE SITUATION DENTAIRE**
VORRICHTUNG ZUR ANALYSE EINER ZAHNSITUATION
DEVICE FOR ANALYSING A DENTAL SITUATION

(43) Date de publication de la demande: 27.11.2019
(73) Titulaire: Dental Monitoring, 75008 Paris (FR)
(72) Inventeur: SALAH, Philippe, 75020 PARIS (FR); PELISSARD, Thomas, 92110 CLICHY (FR); GHYSELINCK, Guillaume, 59169 CANTIN (FR); DEBRAUX, Laurent, 75020 PARIS (FR)
(74) Mandataire: Nony

(56) Documents cités:
- EP-A1- 3 050 512
- WO-A1-2017/182648
- FR-A1- 3 032 282
- US-A1- 2004 197 727
- US-A1- 2017 065 379
- US-A1- 2018 055 600
- US-A1- 2018 110 590

## Description

### Domaine technique

La présente invention concerne un dispositif d'analyse d'une situation dentaire d'un patient.

### Etat de la technique

Lorsqu'un professionnel des soins dentaires intervient sur les dents d'un patient ou qu'il effectue un diagnostic, il a besoin d'évaluer la situation dentaire réelle, et en particulier la position et/ou la forme et/ou les dimensions des dents et/ou le bon positionnement d'un appareil orthodontique fixé sur les dents.

Cette évaluation requiert généralement une comparaison de la situation dentaire réelle avec une situation dentaire théorique. Par exemple, pour diagnostiquer le détachement d'une attache d'un appareil orthodontique, il peut être nécessaire de comparer la situation dentaire réelle dans laquelle l'attache est écartée de manière anormale de la dent sur laquelle elle est supposée être collée, avec une situation dentaire théorique dans laquelle ladite attache est effectivement collée sur ladite dent. Ce besoin existe notamment lors de traitements sur mesure, pour lesquels les attaches sont conformées pour s'adapter précisément à la morphologie des dents et/ou selon les prescriptions spécifiques du professionnel des soins dentaires.

Le professionnel des soins dentaires effectue également une comparaison entre une situation dentaire théorique et une situation dentaire réelle lorsqu'il cherche à évaluer si une dent est anormalement positionnée ou présente une forme anormale. La position ou la forme ou une dimension réelle de la dent doivent en particulier être comparées avec une position ou une forme ou une dimension théorique que la dent devrait présenter au moment où le professionnel des soins dentaires effectue la comparaison.

Par ailleurs, l'analyse de la situation dentaire réelle doit parfois être effectuée très rapidement, voire en temps réel. Par exemple, lorsque le professionnel des soins dentaires intervient au moyen d'un outil pour modifier la forme ou l'apparence d'une dent, l'action qu'il exerce sur l'outil doit être précise et s'adapter en permanence à une situation dentaire réelle qui se modifie.

De manière générale, la qualité de la comparaison entre la situation dentaire réelle et la situation dentaire théorique a un impact sur la qualité du traitement, qu'il soit thérapeutique ou non.

Il existe donc un besoin permanent pour un procédé d'analyse permettant d'améliorer l'appréciation d'une situation dentaire réelle, notamment par un professionnel des soins dentaires.

Par ailleurs, l'efficacité d'un traitement dentaire est directement liée au respect de la prescription médicale par le patient. A défaut d'une bonne observance, la situation dentaire peut empirer, ce qui entraîne un risque supplémentaire pour le patient, mais également des coûts pour la Sécurité Sociale ou les organismes de financement des soins.

La mauvaise observance d'un traitement rend également plus difficiles les études cliniques, en particulier si le patient ne déclare pas les périodes de non observance.

Il existe donc un besoin pour un procédé permettant d'améliorer l'observance.

Un but de l'invention est de répondre, au moins partiellement, à ces besoins.

US 2017/065379 décrit un dispositif pour la conception d'un appareil dentaire, et en particulier d'un dentier. Un modèle 3D comportant un modèle de tête et un modèle d'appareil dentaire positionné dans la bouche du modèle de tête est présenté à l'utilisateur. Ce dernier peut modifier le modèle d'appareil dentaire pour observer les conséquences esthétiques sur le modèle 3D.

US 2018/110590 décrit un procédé destiné à faciliter le positionnement d'un appareil sur une arcade d'un patient. A cet effet, un ordinateur de calcul génère un « modèle de traitement dentaire virtuel » comportant une arcade virtuelle et un appareil virtuel, puis un dispositif de réalité mixte utilise ce modèle pour qu'un praticien puisse simultanément voir les dents du patient et une vue du modèle de traitement dentaire virtuel. Après alignement du modèle de traitement dentaire virtuel avec les dents, le praticien peut ainsi visualiser précisément les emplacements de fixation de l'appareil.

### Résumé de l'invention

L'invention propose un dispositif pour la mise en œuvre d'un procédé d'analyse d'une situation dentaire réelle d'un patient, à des fins de simulation, le procédé comportant une série de cycles d'étapes A) à C), un cycle d'étapes A) à C) comportant les étapes successives suivantes :
A) à un instant actualisé, acquisition d'une image actualisée représentant une scène dentaire réelle telle qu'observée par un opérateur ;
B) détermination d'une scène dentaire virtuelle en fonction de la représentation de la scène dentaire réelle sur l'image actualisée, la scène dentaire virtuelle comportant une représentation de l'arcade du patient et/ou une représentation d'un appareil orthodontique porté par les dents de l'arcade telle qu'anticipée pour un instant de simulation futur, l'instant de simulation étant postérieur à l'instant actualisé de plus de 1 jour ;
C) présentation, sur un écran, de la scène dentaire virtuelle en transparence et en superposition sur la scène dentaire réelle, ou
   affichage de l'image actualisée sur un écran et présentation de la scène dentaire virtuelle en superposition avec la représentation de la scène dentaire réelle sur l'image actualisée affichée sur l'écran, en transparence ou non sur ladite représentation ;
   une étape A) étant reprise moins de 5 secondes après l'étape C),
le dispositif comportant :
- une caméra configurée pour mettre en œuvre l'étape A) ;
- une unité de traitement configurée pour mettre en œuvre l'étape B) ;
- un écran pour mettre en œuvre l'étape C) ;
la caméra, l'unité de traitement et l'écran étant configurés pour communiquer entre eux pour mettre en œuvre lesdites étapes.

Dans un mode de réalisation particulier, l'étape B) consiste en une simulation, pour un instant de simulation, de la scène dentaire réelle représentée sur l'image actualisée, puis en une détermination d'une scène dentaire virtuelle en fonction de ladite simulation.

Comme on le verra plus en détail dans la suite de la description, un dispositif selon l'invention permet donc à l'opérateur d'observer simultanément
- la scène dentaire réelle, directement ou à travers un écran d'une part, ou sur l'image actualisée d'autre part, et
- la scène dentaire virtuelle, en superposition avec la scène dentaire réelle ou avec la représentation de la scène dentaire réelle sur l'image actualisée, respectivement.

Cette superposition facilite considérablement l'analyse, par l'opérateur, de préférence en temps réel, des différences entre la scène dentaire réelle et la scène dentaire virtuelle. Elle permet également d'apporter des informations relatives à la scène dentaire réelle et de les disposer en fonction de la représentation de la scène dentaire réelle sur l'image actualisée. La qualité de la transmission de ces informations à l'opérateur en est améliorée. Enfin, elle permet de simuler de manière réaliste une situation dentaire, par exemple de simuler un traitement esthétique.

Lorsque le dispositif est mis en œuvre par un professionnel des soins dentaires, il lui permet d'améliorer la qualité de son intervention sur le patient. Lorsqu'il est mis en œuvre par le patient notamment, il permet de simuler de manière réaliste l'effet d'un traitement dentaire ou d'une modification d'un traitement dentaire, ce qui améliore l'observance.

Un procédé mis en œuvre par un dispositif selon l'invention peut encore comporter une ou plusieurs des caractéristiques optionnelles suivantes :
- à l'étape B), la scène dentaire virtuelle comporte
   - la représentation d'un ou plusieurs éléments physiques de la scène dentaire réelle modélisés par le modèle de référence, et/ou
   - la représentation d'un ou plusieurs éléments physiques de la scène dentaire réelle non modélisés par le modèle de référence, et/ou
   - la représentation d'un ou plusieurs éléments physiques qui ne sont pas dans la scène dentaire réelle et/ou qui ne sont pas dans le modèle de référence, et/ou
   - un indicateur qui ne représente pas un élément physique de la scène dentaire réelle, de préférence plusieurs tels indicateurs ;
- à l'étape B), on identifie un élément de la scène dentaire virtuelle qui ne respecte pas un critère d'évaluation, et on met en évidence cet élément dans la scène dentaire virtuelle et/ou on ajoute à la scène dentaire virtuelle un message relatif audit élément ;
- le critère d'évaluation est relatif à l'état de santé du patient et/ou au positionnement d'un appareil orthodontique porté par le patient et/ou à l'état d'un appareil orthodontique porté par le patient et/ou à un coût pour un traitement dentaire ;
- le message est une consigne à suivre par le patient et/ou par un professionnel des soins dentaires en charge du patient ;
- à l'étape B), on détermine la nature et/ou l'emplacement et/ou l'apparence d'un élément de la scène dentaire virtuelle, et notamment d'un indicateur, en fonction :
   - du contexte, et en particulier en fonction de la distance à un autre élément de la scène dentaire virtuelle et/ou en fonction d'un objectif poursuivi ;
- à l'étape B), la nature et/ou l'emplacement et/ou l'apparence d'éléments de la scène dentaire virtuelle sont déterminés en fonction de la nature et/ou de l'emplacement et/ou de l'apparence d'éléments affichés dans des scènes dentaires virtuelles antérieures, c'est-à-dire de cycles d'étapes A) à C) antérieurs, et/ou en fonction de l'intervalle temporel avec lesdites scènes dentaires virtuelles antérieures, et/ou en fonction de différences entre les images actualisées de cycles successifs des étapes A) à C) ;
- à l'étape B), on détermine la scène dentaire virtuelle de manière qu'elle comporte des informations, notamment textuelles ou graphiques, sur
   - des différences entre l'image actualisée et l'image de référence, et/ou
   - des différences entre le modèle de référence et la scène dentaire réelle, et/ou
   - un emplacement cible, notamment pour un outil ou un appareil orthodontique ou un article décoratif ;
- à l'étape C), on présente la scène dentaire virtuelle sur la scène dentaire réelle ou sur l'image actualisée de manière que les représentations d'éléments physiques réels de la scène dentaire virtuelle se superposent, de manière réaliste, avec lesdits éléments physiques réels ou avec les représentations desdits éléments physiques réels sur l'image actualisée, respectivement ;
- après l'étape C), on modifie un appareil orthodontique et/ou on agit sur l'arcade et/ou on positionne un article ou un appareil orthodontique en fonction de la présentation de la scène dentaire virtuelle sur la scène dentaire réelle ou sur la représentation de la scène dentaire sur l'image actualisée.

Selon un aspect de l'invention, à l'étape C), la scène dentaire virtuelle est présentée sur un écran transparent, en superposition avec la scène dentaire réelle qu'un opérateur peut observer à travers l'écran, en particulier lorsqu'il est face à l'écran.

De préférence, l'écran et la caméra sont immobilisés par rapport à l'opérateur. De préférence, ils sont intégrés dans des lunettes. Le dispositif selon l'invention est alors particulièrement utile pour qu'un professionnel des soins dentaires portant les lunettes puisse évaluer, en temps réel, la situation dentaire d'un patient qu'il examine.

Notamment suivant cet aspect de l'invention, un dispositif selon l'invention peut être utilisé pour évaluer en temps réel une opération d'abrasion ou de fraisage, ou une opération de mise en position d'un appareil orthodontique ou d'une partie d'un appareil orthodontique.

Selon un aspect de l'invention, à l'étape C), la scène dentaire virtuelle est présentée, en superposition avec la représentation de la scène dentaire réelle sur l'image actualisée, sur un écran opaque, c'est-à-dire à travers lequel l'opérateur ne peut pas voir.

Dans un mode de réalisation, l'écran est celui d'un téléphone portable, d'une tablette, d'un ordinateur portable, ou d'un casque de réalité virtuelle. L'écran peut être également la glace d'un miroir, de préférence équipé d'au moins une caméra.

De préférence, l'opérateur manipule un téléphone portable, un ordinateur portable, un casque de réalité virtuelle ou un miroir équipé d'au moins une caméra, pour acquérir ladite image actualisée et visualiser la scène dentaire réelle et la scène dentaire virtuelle.

Selon ce mode de réalisation, le professionnel de soins dentaires peut travailler sur l'arcade du patient en regardant l'écran. Il voit alors simultanément sur l'écran les images acquises par la caméra et les informations fournies par la scène dentaire virtuelle. Il peut en particulier travailler « en aveugle » (c'est-à-dire sans voir, directement ou à travers un écran, la scène dentaire réelle), sur des régions de l'arcade qu'il ne peut observer directement mais observables par la caméra, notamment lorsque la caméra est indépendante de l'écran et, en particulier lorsque la caméra peut être introduite dans la bouche du patient.

Dans un mode de réalisation, à l'étape B) ou d), la scène dentaire virtuelle est déterminée en fonction d'une valeur d'au moins un paramètre de traitement modifiable par interaction avec ledit téléphone portable, ledit ordinateur portable, ledit casque de réalité virtuelle, ledit appareil photo ou ledit miroir ou lesdites lunettes, de préférence avec le téléphone portable. De préférence, le paramètre de traitement est relatif à un port d'un appareil orthodontique par le patient et/ou au respect d'une consigne de traitement par le patient.

Un procédé mis en œuvre par un dispositif selon l'invention, notamment suivant cet aspect de l'invention, peut faciliter les décisions du patient, en particulier parce qu'il lui permet, en choisissant l'instant de simulation, de visualiser l'effet, sur son apparence, des différentes options de traitement possibles. Le procédé peut être également un outil pédagogique efficace pour améliorer l'observance.

### Utilisation d'un modèle de référence

Dans un mode de réalisation préféré, on détermine la scène dentaire virtuelle au moyen d'un modèle numérique tridimensionnel modélisant numériquement au moins une arcade du patient, appelé « modèle de référence ».

De préférence, le modèle de référence sert également, à l'étape C), pour positionner la scène dentaire virtuelle par rapport à la scène dentaire réelle ou à sa représentation.

Avantageusement, la composition de la scène dentaire virtuelle peut être ainsi entièrement automatisée.

Le procédé mis en œuvre par un dispositif selon l'invention peut en particulier comporter les étapes successives suivantes :
a) à un instant de référence, génération, de préférence au moyen d'un scanner, d'un modèle de référence modélisant numériquement au moins une arcade dentaire du patient;
b) modification du modèle de référence ;
c) à un instant actualisé, acquisition, de préférence au moyen d'une caméra, d'une image actualisée représentant une scène dentaire réelle;
d) recherche, par observation du modèle de référence, optionnellement modifié, d'une image de référence présentant une concordance maximale avec l'image actualisée, puis détermination, en fonction de l'image de référence, d'une scène dentaire virtuelle ;
e) présentation de la scène dentaire virtuelle
   - en transparence et en superposition sur la scène dentaire réelle, en particulier sur un écran à travers lequel la scène dentaire réelle est visible ou en projection sur la scène dentaire réelle, ou
   - en superposition avec la représentation de la scène dentaire réelle sur l'image actualisée affichée sur un écran, en transparence ou non sur ladite représentation,
   puis reprise à l'étape c).

L'étape c) est un cas particulier d'étape A). Les caractéristiques optionnelles applicables à l'étape A) sont donc applicables à l'étape c).

Les étapes a), b) et d) constituent ensemble un cas particulier d'étape B). Les caractéristiques optionnelles applicables à l'étape B) sont donc applicables aux étapes a), b) et d).

L'étape e) est un cas particulier d'étape C). Les caractéristiques optionnelles applicables à l'étape C) sont donc applicables à l'étape e).

De préférence, à l'étape a), on scanne une arcade du patient ou un modèle physique de ladite arcade alors que ladite arcade ou ledit modèle physique de ladite arcade porte ou ne porte pas un appareil orthodontique, et en particulier alors que ladite arcade ou ledit modèle physique de ladite arcade ne porte pas un appareil orthodontique.

L'instant actualisé peut être différent de l'instant de référence, et en particulier être postérieur de plus de 3 jours à l'instant de référence.

Dans un mode de réalisation, la scène dentaire virtuelle est constituée par l'image de référence.

De préférence, à l'étape d), on détermine la nature et/ou l'emplacement et/ou l'apparence d'un élément de la scène dentaire virtuelle, et notamment d'un indicateur, en fonction :
- de l'image de référence, et/ou
- de différences entre l'image actualisée et l'image de référence, et/ou
- du contexte.

Le modèle de référence peut donner accès à des informations « cachées », c'est-à-dire qui ne sont pas accessibles ou qui sont difficilement accessibles à l'opérateur. Ces informations cachées peuvent en particulier dépendent de l'image de référence, et donc de l'image actualisée. Dans un mode de réalisation, au moins une partie des informations cachées sont sélectionnées à l'étape d). L'étape d) permet ainsi de créer une scène dentaire virtuelle qui contient de telles informations et l'étape e) permet de les présenter sur la scène dentaire réelle ou sur sa représentation.

### Applications

Un dispositif selon l'invention peut être en particulier utilisé à des fins non thérapeutiques, en particulier à des fins de recherche, par exemple pour évaluer l'efficacité d'un traitement ou d'un appareil orthodontique, ou à des fins esthétiques, ou à des fins pédagogiques.

La scène dentaire virtuelle est relative à la scène dentaire réelle telle que simulée à l'instant de simulation. En particulier, la scène dentaire virtuelle peut simuler la scène dentaire réelle à l'instant de simulation. La simulation est de préférence obtenue au moyen d'un ordinateur, de préférence au moyen d'un algorithme d'intelligence artificielle.

Le dispositif selon l'invention est alors particulièrement utile pour qu'un opérateur, et en particulier un patient, puisse visualiser une situation dentaire à un instant de simulation postérieur à l'instant actualisé, ou visualiser l'évolution d'une telle situation dentaire en faisant varier l'instant de simulation.

Le procédé comprend une série de cycles d'étapes A) à C), ou spécifiquement, d'étapes c) à e), la scène dentaire virtuelle étant déterminée, à chaque cycle, pour simuler une situation dentaire à l'instant de simulation en cours.

Dans un mode de réalisation, on détermine la scène dentaire virtuelle au moyen d'un outil de simulation dynamique configuré pour fournir au moins un élément de la scène dentaire virtuelle en fonction d'un instant de simulation déterminé postérieur à l'instant actualisé.

Lorsqu'il est utilisé à des fins de simulation, un dispositif selon l'invention peut comporter une ou plusieurs des caractéristiques optionnelles suivantes :
- l'instant de simulation est postérieur à l'instant actualisé de plus de 10 jours ou 100 jours à l'instant actualisé ;
- l'instant de simulation est déterminé avant l'étape A) ou avant l'étape B), de préférence par interaction de l'opérateur avec un écran ;
- ledit écran
   - est un écran tactile et on modifie l'intervalle temporel entre l'instant actualisé et l'instant de simulation par interaction, de préférence par glissement, d'un doigt sur ledit écran, et/ou
   - comporte un champ de saisie de l'instant de simulation ;
- entre deux cycles d'étapes A) à C), on modifie l'intervalle temporel entre l'instant actualisé et l'instant de simulation ;
- on règle l'instant de simulation en modifiant la position d'un curseur représenté sur l'écran ;
- à l'étape B), on détermine la nature et/ou l'emplacement et/ou l'apparence d'un élément de la scène dentaire virtuelle, et notamment d'un indicateur, en fonction :
   - du contexte, et en particulier en fonction de la distance à un autre élément de la scène dentaire virtuelle et/ou en fonction d'un objectif poursuivi, et/ou en fonction de l'instant de simulation ;
- à l'étape B), on détermine, pour l'instant de simulation, une couleur et/ou une forme et/ou une position d'une partie visible ou invisible de la bouche, de préférence d'une mâchoire, d'une couronne dentaire et/ou d'une racine dentaire et/ou de la gencive du patient, et on détermine la scène dentaire virtuelle de manière qu'elle reproduise ladite couleur et/ou forme et/ou position ;
- le patient acquiert ladite image actualisée à l'étape A), et observe l'écran à l'étape C).

Un tel dispositif selon l'invention peut être en particulier utilisé pour simuler :
- l'effet d'un ou plusieurs appareils orthodontiques sur les dents du patient, notamment afin de choisir celui qui lui convient le mieux ;
- l'effet d'un arrêt, temporaire ou définitif, d'un traitement en cours ;
- l'effet d'une application d'une consigne.

Notamment, le dispositif peut être utilisé, notamment à des fins pédagogiques, pour visualiser l'effet d'une modification de la fréquence et/ou de la durée et/ou de la technique de brossage des dents, ou l'effet d'un retard dans un changement de gouttière orthodontique et/ou d'un retard pour prendre rendez-vous chez le professionnel des soins dentaires.

Par exemple, il peut être utilisé pour simuler, à l'instant de simulation, la couleur et/ou la position des dents du patient. Notamment, il peut être utilisé pour visualiser l'effet d'un traitement de blanchiment des dents ou l'effet de la cigarette.

Lorsque le procédé comporte les étapes a) à e), à l'étape b), le modèle de référence peut être modifié pour simuler l'effet de l'écoulement du temps entre l'instant de référence et l'instant de simulation, et en particulier l'effet d'un traitement dentaire, thérapeutique ou non, entre ces instants.

Un dispositif selon l'invention peut être encore notamment utilisé pour :
- évaluer une différence relative à la position et/ou la forme et/ou une dimension d'une ou plusieurs dents et/ou d'un appareil orthodontique entre l'instant de référence et l'instant actualisé, ou entre l'instant de simulation et l'instant actualisé, notamment dans le cadre d'un traitement orthodontique, et/ou
- indiquer un emplacement cible de l'arcade dentaire, en particulier sur une ou plusieurs dents.

Un dispositif selon l'invention peut être en particulier utilisé pour visualiser une abrasion subie par une dent ou une déformation de la gencive entre les instants de référence et actualisé ou entre l'instant de simulation et l'instant actualisé, ou guider la mise en place d'un appareil orthodontique ou d'une partie d'un appareil orthodontique ou d'un implant dentaire ou d'un article décoratif.

L'instant de simulation peut être par exemple écarté de l'instant actualisé de moins de 1 mois, moins de 2 semaines ou moins d'une semaine.

Un dispositif selon l'invention comporte optionnellement des moyens pour générer et optionnellement modifier un modèle de référence aux étapes a) et b), ces moyens comportant de préférence un scanner et un ordinateur.

La caméra peut être configurée pour mettre en œuvre l'étape c).

L'unité de traitement peut être configurée pour mettre en œuvre l'étape d).

Les moyens de présentation peuvent être configurés pour mettre en œuvre l'étape e).

De préférence, la caméra est configurée de manière à acquérir une succession d'images actualisées de la scène dentaire réelle et transmettre lesdites images actualisées à l'unité de traitement.

De préférence,
- l'unité de traitement est configurée pour :
   - rechercher, pour chaque image actualisée reçue de la caméra, par observation d'un modèle de référence, une image de référence présentant une concordance maximale avec ladite image actualisée,
   - déterminer, en fonction de l'image de référence, une scène dentaire virtuelle, et
   - transmettre ladite scène dentaire virtuelle aux moyens de présentation ;
- les moyens de présentation sont configurés pour faire apparaitre la scène dentaire virtuelle en superposition sur la scène dentaire réelle ou sur la représentation de la scène dentaire réelle dans l'image actualisée.

Dans un mode de réalisation, la scène dentaire virtuelle est présentée en transparence sur la scène dentaire réelle ou sur sa représentation dans l'image actualisée.

Dans un mode de réalisation, le dispositif comporte des lunettes dans lesquelles la caméra et les moyens de présentation, de préférence la caméra, les moyens de présentation et l'unité de traitement sont intégrés.

Dans un mode de réalisation, les moyens de présentation sont configurés pour présenter la scène dentaire virtuelle sur un verre des lunettes, en superposition réaliste avec la scène dentaire réelle.

Dans un mode de réalisation, le dispositif comporte un téléphone portable ou un appareil photo ou un ordinateur portable ou un casque de réalité virtuelle ou une tablette ou un miroir, dans lequel la caméra et les moyens de présentation, de préférence la caméra, les moyens de présentation et l'unité de traitement sont intégrés.

Les moyens de présentation comportent de préférence un écran permettant d'afficher l'image actualisée et de présenter la scène dentaire virtuelle sur la représentation de la scène dentaire réelle, en « réalité augmentée ».

La représentation de la scène dentaire réelle peut être une image affichée sur l'écran du téléphone portable ou de l'appareil photo ou de l'ordinateur portable ou du casque de réalité virtuelle ou de la tablette.

La représentation de la scène dentaire réelle peut être le reflet renvoyé par le miroir.

Comme on le verra plus en détail dans la suite de la description, l'observateur peut ainsi observer la scène dentaire réelle ou sa représentation sur l'image actualisée et la scène dentaire virtuelle, de préférence en transparence, sur la scène dentaire réelle ou sur sa représentation sur l'image actualisée. Il peut ainsi immédiatement avoir accès aux informations contenues dans la scène dentaire virtuelle, présentées dans l'environnement de la scène dentaire réelle. L'intégration de l'écran dans des lunettes est particulièrement avantageuse à cet effet.

### Définitions

Un « patient » est une personne pour laquelle un procédé mis en œuvre par un dispositif selon l'invention est mis en œuvre, indépendamment du fait que cette personne suive un traitement dentaire ou non.

Une « situation dentaire » définit un ensemble de caractéristiques relatives à une arcade d'un patient à un instant, par exemple la position des dents, leur forme, la position d'un appareil orthodontique, etc. à cet instant.

Par « professionnel des soins dentaires », on entend toute personne qualifiée pour prodiguer des soins dentaires, ce qui inclut un orthodontiste et un dentiste. L'opérateur qui porte les lunettes est de préférence un orthodontiste ou un dentiste.

Par « opérateur », on entend la personne qui met en œuvre le procédé pour que lui soit présentée la scène dentaire virtuelle à l'étape C). L'opérateur peut être en particulier le patient ou le professionnel de soins dentaires.

Par « lunettes », on entend un appareil qui peut être porté devant les yeux d'un opérateur, de préférence en prenant appui sur le nez et/ou les oreilles de l'opérateur.

Un « appareil orthodontique » est un appareil porté ou destiné à être porté par un patient. Un appareil orthodontique peut être destiné à un traitement thérapeutique ou prophylactique, mais également à un traitement esthétique. Un appareil orthodontique peut être en particulier un appareil à arc et attaches, ou une gouttière orthodontique. Une telle gouttière s'étend de manière à suivre les dents successives de l'arcade sur laquelle elle est fixée. Elle définit une goulotte de forme générale en « U ». La configuration d'un appareil orthodontique peut être en particulier déterminée pour assurer sa fixation sur les dents, mais également en fonction d'un positionnement cible souhaité pour les dents. Plus précisément, la forme est déterminée de manière que, dans la position de service, l'appareil orthodontique exerce des contraintes tendant à déplacer les dents traitées vers leur positionnement cible (appareil orthodontique actif), ou à maintenir les dents dans ce positionnement cible (appareil orthodontique passif, ou « de contention »).

Par « modèle », on entend un modèle tridimensionnel numérique.

Par "image", on entend une image en deux dimensions. Une image est formée de pixels. Les « images actualisées » sont des images prises à un instant dit « actualisé ». Elles sont de préférence extraites d'un film pris par une caméra. Une « image de référence » est une vue d'un modèle « de référence » qui présente une concordance maximale avec une image actualisée.

Par « arcade », on entend tout ou partie d'une arcade dentaire. Par « image d'une arcade », ou « modèle d'une arcade », on entend ainsi une représentation en 2 ou 3 dimensions, respectivement, de tout ou partie de ladite arcade.

Une «scène» est constituée par un ensemble d'éléments qui peuvent être observés simultanément. Une « scène dentaire » est une scène comportant une arcade.

Une « scène réelle » est constituée d'éléments physiques. Une scène dentaire réelle comporte donc une arcade, mais aussi, optionnellement, un appareil orthodontique porté par les dents de l'arcade ou que l'opérateur manipule, et/ou un outil qu'un opérateur manipule.

Une «scène virtuelle» est constituée de représentations d'éléments, notamment des représentations
- d'un élément physique de la scène dentaire réelle correspondante (c'est-à-dire de la scène dentaire réelle représentée sur l'image actualisée à partir de laquelle la scène dentaire virtuelle a été déterminée) ou
- d'une autre information, ou « indicateur ».

Un élément physique peut être modélisé par le modèle de référence, par exemple un contour de dent, ou non modélisé par le modèle de référence, par exemple un outil manipulé par l'opérateur.

Une représentation d'un élément physique n'est pas nécessairement réaliste. Par exemple, un outil peut être symbolisé par une droite.

La présentation d'une scène dentaire virtuelle est « en transparence » sur une scène dentaire réelle lorsqu'elle permet à l'opérateur d'apercevoir, à travers ladite scène dentaire virtuelle, la scène dentaire réelle.

La présentation d'une scène dentaire virtuelle est « en transparence » sur une image actualisée représentant une scène dentaire réelle lorsqu'elle permet à l'opérateur d'apercevoir l'image actualisée à travers ladite scène dentaire virtuelle.

Une scène dentaire virtuelle est affichée « en superposition » ou « en registre » avec une scène dentaire réelle ou avec une représentation d'une scène dentaire réelle sur une image actualisée lorsqu'elle comporte des représentations d'éléments physiques de la scène dentaire réelle qui sont présentées de manière que les contours desdites représentations, éventuellement transparents, se superposent aux contours desdits éléments physiques ou desdites représentations desdits éléments physiques sur l'image actualisée, respectivement. La superposition apparaît ainsi réaliste. Par exemple, lorsque l'observateur observe une dent d'une arcade dentaire, la présentation est déterminée de manière que la représentation de la dent sur la scène dentaire virtuelle vienne se superposer à la vue de cette dent perçue par l'opérateur.

« L'emplacement » d'un élément de la scène dentaire virtuelle fait référence à sa position par rapport aux éléments de la scène dentaire réelle ou de la représentation de ces éléments sur l'image actualisée lorsque la scène dentaire virtuelle est présentée à l'étape C).

On appelle « concordance » (*« match »* ou *« fit » en* anglais) entre deux objets une mesure de la différence, ou « distance », entre ces deux objets.

De préférence, deux images ou « vues » qui présentent une concordance maximale représentent sensiblement les mêmes éléments de la même façon. Autrement dit, les représentations des éléments sur ces deux images sont sensiblement superposables.

Il faut interpréter "comprendre " ou "comporter " ou "présenter " ou « représenter» de manière non restrictive, sauf indication contraire.

### Brève description des figures

D'autres caractéristiques et avantages de l'invention apparaîtront encore à la lecture de la description détaillée qui va suivre et à l'examen du dessin annexé dans lequel :
- la figure 1 représente, schématiquement, les différentes étapes d'un procédé mis en œuvre par un dispositif selon un mode de réalisation préféré de l'invention ;
- la figure 2 représente un exemple d'une image de référence d'un modèle de référence ;
- les figures 3 (3a-3b) et 4 (4a et 4b) illustrent le traitement d'une image de référence et d'une image actualisée, respectivement, pour en extraire une carte de référence et une carte actualisée, respectivement, représentant une information de contour ;
- les figures 5, 6 et 7 illustrent schématiquement des exemples de dispositifs selon l'invention ;
- la figure 8 représente la superposition d'une scène dentaire virtuelle sur une image actualisée.

Des références identiques sont utilisées pour désigner des organes identiques ou analogues sur les différentes figures.

### Description détaillée

### Dispositif

Un dispositif 10 selon l'invention comporte une caméra 16, une unité de traitement informatique 12 et des moyens de présentation 18. La caméra 16, l'unité de traitement 12 et les moyens de présentation sont pourvus de moyens leur permettant de communiquer entre eux.

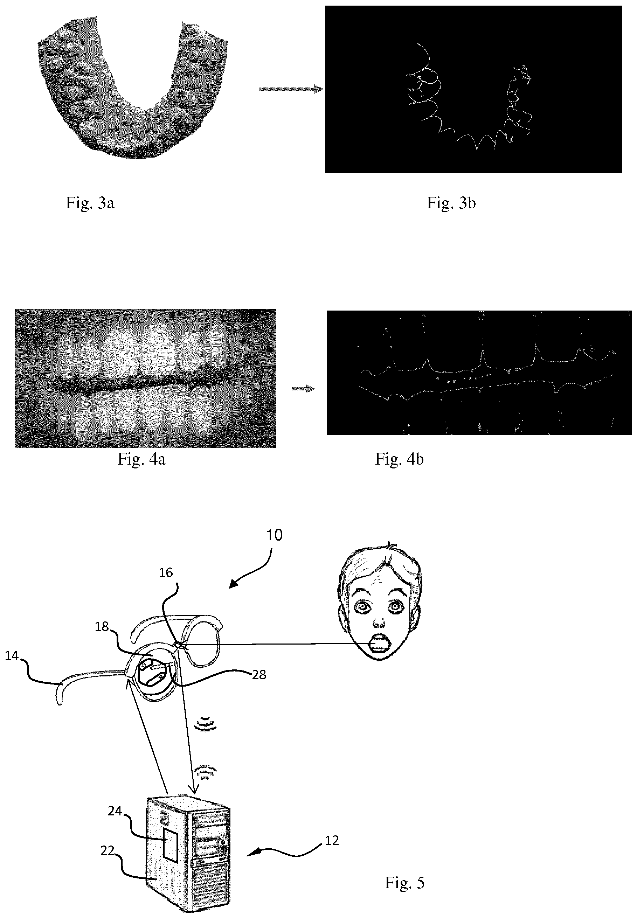

Comme représenté sur les figures 5, 6 et 7, la caméra 16 et/ou l'unité de traitement 12 et/ou les moyens de présentation peuvent être intégrés dans des lunettes comportant classiquement une monture 14 configurée pour être portée par le nez et les oreilles d'un opérateur, dans un téléphone portable, dans une tablette, dans un ordinateur portable, dans un casque de réalité virtuelle, dans un appareil photo ou dans un miroir 17.

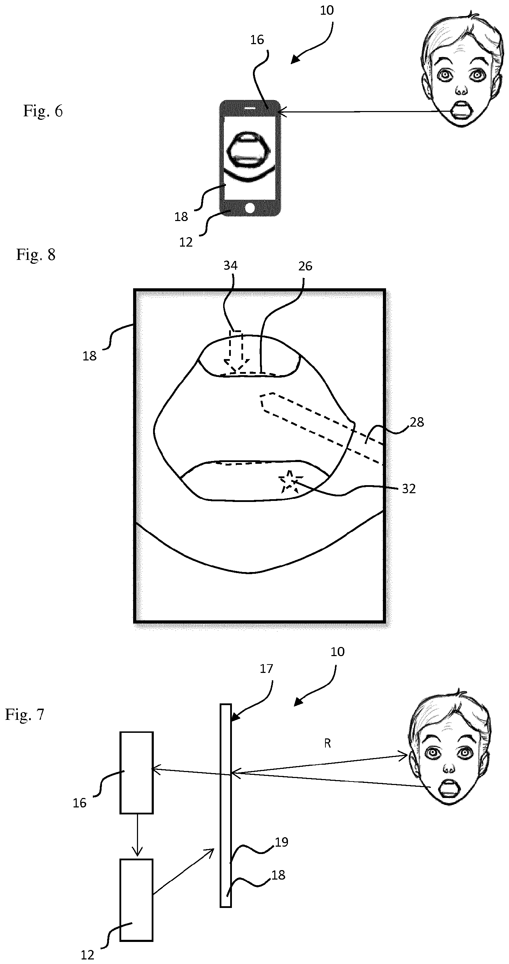

La caméra 16 est destinée à l'acquisition d'images actualisées représentant la scène dentaire réelle observée par l'opérateur à travers les lunettes (figure 5) ou sur l'écran d'un téléphone portable (figure 6) ou d'un ordinateur portable, d'un casque de réalité virtuelle, d'un appareil photo ou d'une tablette, ou sur la glace 19 du miroir (figure 7). Elle peut être une caméra classique.

Dans un mode de réalisation, le dispositif comporte des moyens de projection de motifs de lumière structurée, par exemple d'un nuage de points, par exemple laser, en particulier infrarouge. Avantageusement, la scène dentaire réelle fait apparaître ces motifs de manière déformée, ce qui permet d'en déduire des informations relatives à la profondeur.

Dans le cas où le dispositif comporte un miroir, la glace 19 peut être une glace sans tain et la caméra peut être disposée derrière la glace, comme sur la figure 7.

Dans un mode de réalisation, le dispositif comporte plusieurs caméras, ce qui permet, par simple calcul trigonométrique, d'évaluer la distance entre les objets observés et la caméra. La multiplicité des caméras permet ainsi d'accélérer la recherche des images de référence. Les caméras permettent ainsi également de simuler la vue tridimensionnelle qu'a l'observateur de la scène dentaire réelle.

Par « moyens de présentation », on entend tout moyen configuré pour recevoir, de l'unité de traitement 12, une scène dentaire virtuelle et faire apparaître ladite scène dentaire virtuelle sur la scène dentaire réelle, sur un écran à travers lequel un observateur peut voir la scène dentaire réelle ou sur l'image actualisée.

Dans un mode de réalisation, les moyens de présentation 18 comportent un écran et un projecteur pour projeter sur l'écran la scène dentaire virtuelle.

En particulier dans le mode de réalisation dans lequel la caméra est intégrée dans des lunettes, l'écran peut être transparent. Il est de préférence fixé sur la monture 14, et la scène dentaire virtuelle est présentée sur l'écran afin que l'opérateur voie, en observant cet écran, à la fois la scène dentaire réelle à travers l'écran, mais aussi la scène dentaire virtuelle projetée sur l'écran.

Les lunettes comportent classiquement deux verres qui s'étendent devant les deux yeux de l'opérateur, respectivement, mais le nombre de verres n'est pas limitatif. Les verres peuvent être correcteurs de la vision ou non. Plusieurs verres peuvent s'étendre devant un même œil. Par exemple, un premier verre peut corriger la vue de l'opérateur et un deuxième verre peut être un verre servant d'écran.

L'appareil HoloLens, développé par la société Microsoft^{®}, est un exemple de moyens de présentation.

L'écran peut être opaque et l'image actualisée et la scène dentaire virtuelle sont présentées sur l'écran, en registre, afin que l'opérateur voie, en observant cet écran, à la fois l'image actualisée représentant la scène dentaire réelle, mais aussi la scène dentaire virtuelle.

Dans ce mode de réalisation notamment, la caméra peut être immobilisée par rapport à l'écran, par exemple quand la caméra et l'écran sont intégrés dans un téléphone portable (figure 6) ou un ordinateur portable ou un casque de réalité virtuelle ou un appareil photo ou une tablette ou un miroir

Alternativement, la caméra peut être libre (ou «indépendante») par rapport à l'écran. Avantageusement, ce dernier mode de réalisation permet une grande souplesse. Par exemple, dans un mode de réalisation, la caméra peut être introduite à l'intérieur de la bouche alors que l'écran reste à l'extérieur de la bouche.

En particulier dans le mode de réalisation dans lequel la caméra est intégrée dans un miroir ou est solidaire d'un miroir, l'écran peut être la glace 19 réfléchissante du miroir, et la scène dentaire virtuelle peut être présentée sur l'écran, en registre avec le reflet R renvoyé par la glace 19, afin que l'opérateur voie, en observant cet écran, à la fois le reflet R représentant la scène dentaire réelle, mais aussi la scène dentaire virtuelle superposée à la scène dentaire réelle.

Le reflet, comme l'image actualisée affichée sur l'écran des lunettes, du téléphone portable, de l'ordinateur portable, du casque de réalité virtuelle, de l'appareil photo ou de la tablette, donne ainsi à l'opérateur une représentation de scène dentaire réelle.

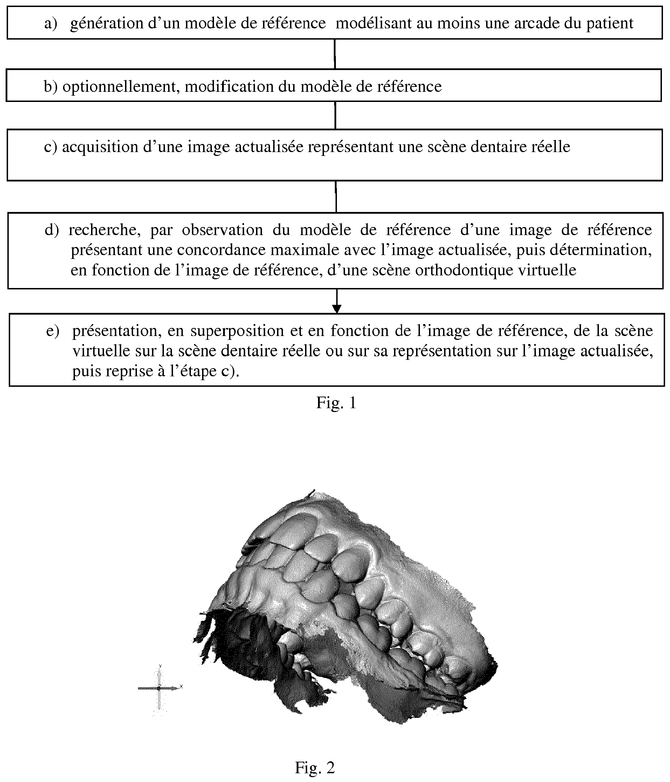

L'unité de traitement 12 peut comporter les moyens électroniques conventionnels de tout ordinateur, et en particulier une unité centrale 22, un programme et une mémoire 24. La mémoire 24 contient de préférence un modèle de référence d'au moins une partie de la scène dentaire réelle, et en particulier un modèle de l'arcade dentaire, par exemple comme représenté sur la figure 2. Le programme comporte des instructions de code permettant, lorsqu'elles sont exécutées, de mettre en œuvre l'étape d), et en particulier d'analyser une image actualisée, de rechercher une vue du modèle de référence qui présente une concordance maximale avec l'image actualisée, c'est-à-dire une image de référence, puis de déterminer, en conséquence, une scène dentaire virtuelle.

Dans un mode de réalisation, le dispositif comporte encore une interface permettant à un opérateur de paramétrer la scène dentaire virtuelle. De préférence, l'interface est configurée pour qu'un opérateur puisse modifier un instant de simulation déterminant la scène dentaire virtuelle. Le contenu de la scène dentaire virtuelle peut en particulier être constitué par la représentation d'éléments de la scène dentaire réelle sous une apparence et/ou à une position simulées pour l'instant de simulation passé ou futur ou connue à un instant de simulation passé.

Par exemple, dans un mode de réalisation, l'écran est un écran tactile et l'opérateur peut modifier l'instant de simulation en entrant une date dans un champ de saisie ou en déplaçant un ou plusieurs doigts sur l'écran. Par exemple un déplacement vers la droite de l'écran peut conduire à avancer vers le futur et un déplacement vers la gauche de l'écran peut conduire à reculer dans le passé. La scène dentaire virtuelle présentée s'adapte en conséquence, de préférence en temps réel.

L'interface peut être également un organe mécanique, par exemple un bouton ou une mollette.

Dans un mode de réalisation, le dispositif est configuré pour que l'intervalle de temps entre l'instant actualisé et l'instant de simulation évolue continûment d'un cycle à l'autre, c'est-à-dire que la succession des cycles d'étapes A) à C) ou c) à e) simule un avancement dans le temps ou un retour dans le temps. Dans un mode de réalisation, l'écart temporel entre deux instants de simulation successifs est constant, ce qui permet de visualiser une évolution dans laquelle le temps s'écoule à vitesse constante.

L'unité de traitement détermine les conditions dans lesquelles la scène dentaire virtuelle doit être présentée pour apparaître, à l'opérateur, en superposition avec la scène dentaire réelle.

Lorsque la scène dentaire virtuelle doit être présentée sur l'image actualisée affichée sur un écran, l'opérateur observe l'écran et voit donc ce qui est observé par la caméra. De simples opérations, comme une mise à l'échelle, une correction des effets de perspective ou un recalage sur l'image actualisée, peuvent suffire pour assurer une superposition réaliste de la scène dentaire virtuelle sur l'image actualisée, par exemple en utilisant des marques caractéristiques, comme décrit ci-après.

Lorsque la scène dentaire virtuelle doit être présentée sur un écran transparent à travers lequel un opérateur observe la scène dentaire réelle, il est préférable que la caméra soit disposée de manière à capturer une image actualisée qui corresponde à la vue de la scène dentaire réelle par l'observateur. Il est également préférable que l'écran soit à une distance constante, et orienté de manière fixe par rapport à l'observateur. Avantageusement, l'unité de traitement peut ainsi déterminer facilement, à partir de l'image actualisée acquise par la caméra, la vue de la scène dentaire réelle observée par l'opérateur, ainsi que les conditions dans lesquelles la scène dentaire virtuelle doit être présentée pour apparaître, à l'opérateur, en superposition avec la scène dentaire réelle.

### Procédé

On décrit un procédé pouvant être mis en œuvre au moyen d'un dispositif selon l'invention.

**A l'étape a),** à un instant de référence, on génère un modèle de référence, de préférence au moyen d'un scanner.

Le modèle de référence peut être un modèle de l'arcade du patient ou être composé d'un modèle de cette arcade et d'un modèle d'un appareil orthodontique disposé sur ledit modèle de l'arcade.

La création du modèle de référence résulte de préférence d'une prise de mesures.

De préférence, on scanne une arcade du patient avec un scanner 3D, optionnellement alors que le patient porte un appareil orthodontique, ou un moulage de cette arcade, de manière à créer un modèle de référence. De préférence, le scan est réalisé avec un scanner 3D classique.

Le modèle de référence obtenu au moyen d'un scanner représente une arcade, mais également, le cas échéant, tout objet scanné en même temps que l'arcade, par exemple un appareil orthodontique ou un article décoratif fixé sur l'arcade.

Le modèle de référence peut être alternativement un modèle générique d'arcade, choisi dans une base de modèles génériques, c'est-à-dire un modèle d'arcade applicable, de manière grossière, à plusieurs patients, ou résulter d'une déformation d'un tel modèle générique. La déformation peut en particulier être en fonction de mesures ou d'observations faites sur le patient, par exemple en fonction de photos de ses arcades dentaires. La déformation d'un modèle afin qu'il puisse correspondre à un ou plusieurs photos peut mettre en œuvre une méthode métaheuristique, comme décrit dans WO 2016/066651 notamment.

Un modèle générique d'arcade peut être en particulier un modèle obtenu par un traitement statistique, par exemple une moyenne, sur plus de 5, plus de 10, plus de 100 ou plus de 1000 modèles historiques générés au moyen d'un scanner. Les modèles historiques sont de préférence des modèles d'arcades dentaires de patients « historiques » ayant des caractéristiques dentaires identiques ou similaires à celles du patient pour lequel on génère le modèle de référence, par exemple ayant souffert de la même pathologie et/ou ayant le même âge et/ou ayant le même sexe et/ou ayant une morphologie similaire.

Le modèle de référence, tridimensionnel, peut être observé selon un angle quelconque. Une observation du modèle, selon un angle et à une distance déterminés, est une « vue du modèle ». Les figures 2 et 3a sont des exemples de vues d'un modèle de référence.

Les vues du modèle de référence sont destinées à être comparées avec les images actualisées afin de déterminer, pour chaque image actualisée, une image de référence la plus ressemblante possible à l'image actualisée, puis constituer en conséquence une scène dentaire virtuelle apportant des informations relatives aux éléments représentés sur l'image actualisée.

L'instant de référence peut être proche de l'instant actualisé, par exemple éloigné (postérieur ou antérieur) de moins de 1 mois, 2 semaines ou 1 semaine ou 3 jours à l'instant actualisé. Des images actualisées peuvent alors être utilisées pour déformer un modèle générique jusqu'à obtenir le modèle de référence correspondant à la situation dentaire à l'instant actualisé. Par ailleurs, la scène dentaire virtuelle apporte alors des informations sur la situation dentaire propres à l'instant actualisé. Par exemple, si le modèle de référence modélise les racines des dents, la scène dentaire virtuelle peut représenter les racines des dents.

L'instant de référence peut être éloigné de l'instant actualisé, par exemple éloigné de plus de 1 mois à l'instant actualisé, 2 semaines ou 1 semaine ou 3 jours. Le modèle de référence peut alors différer sensiblement de la réalité à l'instant actualisé. Les informations fournies par la scène dentaire virtuelle peuvent alors porter sur l'évolution de la situation dentaire entre l'instant de référence et l'instant actualisé. Par exemple, elles peuvent renseigner sur un déplacement de dents ou d'un appareil orthodontique.

Par exemple, le procédé peut être utilisé pour contrôler si un appareil de contention passif maintient bien les dents en position après un traitement dentaire. Le modèle de référence peut être généré immédiatement après la pose de l'appareil et l'acquisition d'une image actualisée peut avoir lieu un mois après cette pose. L'image de référence et la scène dentaire virtuelle concerneront donc les dents dans leur position en fin de traitement et la présentation de la scène dentaire virtuelle sur la scène dentaire réelle, à l'instant actualisé, permettra ainsi au professionnel des soins dentaires de détecter les différences avec leurs positions réelles.

**A l'étape b),** dans un mode de réalisation, le modèle de référence est modifié.

La modification peut être réalisée à tout moment entre l'instant de référence et l'instant actualisé. De préférence, elle est effectuée sensiblement à l'instant de référence.

La modification du modèle de référence peut être une déformation de ce modèle.

Dans un mode de réalisation, le modèle de référence est segmenté, et en particulier des modèles de dent sont créés, comme décrit dans WO 2016/066651. Puis les modèles de dent sont déplacés ou déformés.

La modification à l'étape b) peut simuler l'effet de l'écoulement du temps, par exemple l'effet d'un traitement dentaire, entre l'instant de référence et l'instant actualisé. La modification du modèle de référence conduit ainsi à un modèle représentant les dents dans une position ou une forme estimée pour l'instant actualisé.

Le déplacement ou la déformation des modèles de dent peuvent être également déterminés ou affinés à partir d'une ou plusieurs images actualisées, comme décrit dans WO 2016/066651. De préférence, le modèle de référence initial, déterminé à l'étape a), résulte alors de mesures au moyen d'un scan d'au moins une arcade ou d'au moins un moulage d'une arcade.

Le procédé peut être ainsi utilisé pour vérifier si le déroulement d'un traitement dentaire est conforme aux prévisions, le modèle de référence initial étant modifié pour correspondre à la situation anticipée à l'instant actualisé. En orthodontie, les évolutions sensibles peuvent prendre quelques jours. De préférence, dans ce mode de réalisation, l'intervalle de temps entre l'instant actualisé et l'instant de référence est supérieur à 3 jours, voire supérieur à 1 semaine, supérieur à 2 semaines, supérieur à 4 semaines, supérieur à 8 semaines, supérieur à 12 semaines, voire supérieur à 16 semaines.

L'image de référence, résultant d'une observation du modèle de référence modifié fait alors apparaitre les dents dans leurs positions ou formes anticipées à l'instant actualisé. Elle constitue ou peut être intégrée dans une scène dentaire virtuelle dont la présentation, à l'étape e), permet au professionnel des soins dentaires de détecter les différences entre les positions ou formes réelles des dents et les positions ou formes anticipées pour l'instant actualisé.

La modification du modèle de référence initial peut consister en un ajout d'un autre modèle et/ou dans la modification de cet autre modèle. Par exemple, un modèle d'un appareil orthodontique posé sur l'arcade peut être ajouté à un modèle de référence qui ne représenterait initialement que l'arcade. La modification du modèle de référence peut alors inclure une modification de cet appareil orthodontique pour qu'il apparaisse dans sa position ou forme anticipée pour l'instant actualisé. Sa présentation, à l'étape e), permet alors au professionnel des soins dentaires de détecter les différences entre la position ou forme réelle de l'appareil orthodontique et la position ou forme anticipées pour l'instant actualisé, et notamment de détecter tout décollement d'une attache.

**A l'étape c)**, à l'instant actualisé, on acquiert avec la caméra une image actualisée représentant une scène dentaire réelle. Sur les figures, l'image actualisée est représentée en trait continu sur l'écran 18.

Un dispositif selon l'invention dans lequel la caméra est fixée sur la monture de lunettes ou est intégrée dans un miroir permet d'acquérir une image actualisée qui représente avec une grande précision la scène dentaire réelle observée par l'opérateur à travers les verres de ces lunettes ou sur ce miroir.

Un dispositif selon l'invention dans lequel la caméra est intégrée dans un téléphone portable, un ordinateur portable, une tablette ou un appareil photo permet d'acquérir une image actualisée qui représente exactement la scène dentaire réelle observée par l'opérateur sur l'écran du téléphone portable, de l'ordinateur, de la tablette, ou de l'appareil photo respectivement.

Avec un casque de réalité virtuelle notamment, la caméra peut être rigidement solidaire ou être indépendante de l'écran. L'indépendance de la caméra et de l'écran permet avantageusement à l'opérateur d'opérer sans voir directement la scène dentaire réelle, c'est-à-dire en ne la voyant que sur l'écran. Il peut ainsi travailler « en aveugle ».

De préférence, l'image actualisée est en couleurs, de préférence en couleurs réelles.

**A l'étape d),** on recherche une image de référence qui présente une concordance maximale avec l'image actualisée.

A l'étape d), toutes les méthodes connues pour réaliser une concordance maximale sont envisagées.

L'obtention d'une concordance maximale peut notamment résulter d'une optimisation de manière à minimiser ladite distance (« *best fit »*)*.*

De préférence, l'image de référence est recherchée en observant le modèle de référence selon différents angles d'observation et différentes distances, jusqu'à obtenir une vue du modèle de référence qui corresponde sensiblement à l'image actualisée, c'est-à-dire qui puisse lui être sensiblement superposée. Pour chaque image actualisée, on obtient ainsi une image de référence présentant une concordance maximale avec l'image actualisée. Cette recherche est de préférence effectuée au moyen d'une méthode métaheuristique, de préférence évolutionniste, de préférence par recuit simulé.

Les méthodes métaheuristiques sont des méthodes d'optimisation connues. Elles sont de préférence choisies dans le groupe formé par
- les algorithmes évolutionnistes, de préférence choisie parmi: les stratégies d'évolution, les algorithmes génétiques, les algorithmes à évolution différentielle, les algorithmes à estimation de distribution, les systèmes immunitaires artificiels, la recomposition de chemin Shuffled Complex Evolution, le recuit simulé, les algorithmes de colonies de fourmis, les algorithmes d'optimisation par essaims particulaires, la recherche avec tabous, et la méthode GRASP ;
- l'algorithme du kangourou,
- la méthode de Fletcher et Powell,
- la méthode du bruitage,
- la tunnelisation stochastique,
- l'escalade de collines à recommencements aléatoires,
- la méthode de l'entropie croisée, et
- les méthodes hybrides entre les méthodes métaheuristiques citées ci-dessus.

L'exploration du modèle de référence pour rechercher l'image de référence peut comprendre une ou plusieurs des caractéristiques des étapes c), d) et e) de WO 2016 066651, dans la mesure où elles concernent une telle exploration.

De préférence, on traite l'image actualisée pour réaliser une carte actualisée représentant, au moins partiellement, une information discriminante, et ladite recherche comporte les étapes suivantes :
i) acquisition d'une vue du modèle de référence ;
ii) traitement de la vue pour réaliser au moins une carte de référence représentant, au moins partiellement, l'information discriminante ;
iii) comparaison des cartes actualisée et de référence de manière à déterminer une distance entre lesdites cartes actualisée et de référence et, si la distance est supérieure à un seuil, modification de la vue, puis reprise à l'étape ii).

Les cartes actualisée et de référence représentent la même information discriminante. L'information discriminante est une information caractéristique qui peut être extraite d'une image (*"image feature"*)*,* classiquement par un traitement informatique de cette image. Elle est de préférence choisie dans le groupe constitué par une information de contour, une information de couleur, une information de densité, une information de distance, une information de brillance, une information de saturation, une information sur les reflets et des combinaisons de ces informations.

L'homme de l'art sait comment traiter une image pour faire apparaître l'information discriminante, et donc créer la carte correspondante.

Par exemple, la figure 4b est une carte actualisée relative au contour des dents obtenue à partir de l'image actualisée de la figure 4a. La figure 3b est une carte de référence relative au contour des dents obtenue à partir de la vue de la figure 3 a.

La modification de la vue est de préférence guidée par des règles heuristiques, par exemple en favorisant les modifications qui, d'après une analyse des précédentes distances, apparaissent les plus favorables pour la réduire.

L'étape iii) aboutit à une image de référence sensiblement superposable à l'image actualisée.

L'obtention d'une concordance maximale entre l'image de référence et l'image actualisée peut également résulter de la superposition de repères identifiés sur ces deux images, par exemple la superposition de repères fixes comme l'extrémité d'une dent ou un point de contact entre deux dents.

La scène dentaire virtuelle comporte de préférence, voire est constituée de l'image de référence. L'image de référence est un exemple particulier d'une scène dentaire virtuelle.

Sur la figure 8, les éléments de la scène dentaire virtuelle sont représentés en trait interrompu.

Dans un mode de réalisation préféré, une scène dentaire virtuelle comporte une représentation, de préférence réaliste, d'un ou plusieurs éléments de la scène dentaire réelle correspondante. En particulier, de préférence, elle comporte une représentation de l'arcade, mais aussi optionnellement, par exemple, une représentation d'un appareil orthodontique porté par les dents de l'arcade ou que l'opérateur manipule devant l'arcade, ou d'un outil que l'opérateur manipule devant l'arcade.

Comme représenté sur la figure 8, la scène dentaire virtuelle peut par exemple représenter les contours 26 des dents et sa projection sur les dents peut faciliter la détection de déplacements ou de déformations des dents.

La scène dentaire virtuelle peut également comporter la représentation d'un ou plusieurs éléments physiques de la scène dentaire réelle non modélisés par le modèle de référence, par exemple un outil 28 manipulé par l'opérateur. Pour identifier de tels éléments physiques, l'image actualisée est analysée, par exemple au moyen d'algorithmes d'intelligence artificielle.

La scène dentaire virtuelle peut également comporter la représentation d'un ou plusieurs éléments physiques qui ne sont pas dans la scène dentaire réelle, par exemple un outil ou un appareil orthodontique ou un article décoratif 32, par exemple positionné sur la scène dentaire virtuelle de manière à apparaître, sur la scène dentaire réelle, à un emplacement (« emplacement cible ») où l'outil ou l'appareil orthodontique ou l'article décoratif, respectivement, devrait être disposé par le professionnel des soins dentaires ou par le patient.

La représentation d'un élément physique peut être réaliste, par exemple être constituée des contours de l'élément physique considéré, ou symbolique. Par exemple, le foret d'une perceuse peut être symbolisé par une flèche.

La représentation des éléments physiques de la scène dentaire virtuelle peut être bidimensionnelle et/ou tridimensionnelle. La présentation de la scène dentaire virtuelle peut être en particulier un hologramme.

La scène dentaire virtuelle peut également comporter, en complément ou alternativement à la représentation d'éléments physiques, par exemple de l'image de référence, un ou plusieurs indicateurs 34.

Un indicateur est un élément d'une scène dentaire virtuelle qui ne représente pas, même symboliquement, un élément physique de la scène dentaire réelle.

Un indicateur peut être en particulier un texte ou un symbole (un point, un trait, une flèche, un aplat de couleur...), par exemple un symbole illustrant des différences entre l'image actualisée et l'image de référence, ou fournissant des informations relatives à un acte effectué par l'opérateur, par exemple lui indiquant une direction pour un outil qu'il manipule.

Dans un mode de réalisation, l'indicateur pointe sur un emplacement sur lequel l'opérateur doit apporter un soin particulier, par exemple un brossage. L'indicateur peut être une image, de préférence ludique, par exemple un dessin d'un monstre ou d'un microbe, sur lequel l'opérateur doit appliquer un outil, par exemple une brosse à dent. Un tel indicateur améliore l'apprentissage du brossage des dents, par exemple.

Sur la figure 8, l'indicateur 34 pointe vers une région dentaire qui a été abrasée.

La nature d'un indicateur peut être déterminée en fonction de l'image de référence. Les indicateurs peuvent être en particulier associés au modèle de référence ou à une région du modèle de référence. Ils sont par exemple stockés dans une base de données dans la mémoire 24 de l'unité de traitement.

Par exemple, des informations complémentaires peuvent être associées à différentes régions du modèle de référence, par exemple pour indiquer des caries, des zones fragiles, ou des zones de collage pour un appareil orthodontique. Des informations complémentaires peuvent être également associées au modèle de référence dans son ensemble, par exemple pour préciser les circonstances de sa réalisation ou des informations sur le patient ou sur l'appareil orthodontique éventuellement représenté. Les indicateurs permettent de présenter ces informations complémentaires.

La nature d'un indicateur peut être déterminée en fonction des différences entre l'image actualisée et l'image de référence.

Par exemple, des indicateurs peuvent être ajoutés pour mettre en évidence une déformation ou un déplacement d'une dent, ou un détachement d'un appareil orthodontique.

La nature et/ou l'emplacement et/ou l'apparence d'un indicateur peut être déterminée en fonction du contexte, et en particulier en fonction de la distance à un autre élément de la scène dentaire virtuelle et/ou en fonction d'un objectif poursuivi. Par exemple, il apparaîtra avec une apparence fonction de la distance à une dent particulière ou fonction du risque pour le patient.

Le contexte est défini par les conditions dans lesquelles le procédé est utilisé.

Par exemple, l'opérateur peut avoir pour objectif de vérifier le positionnement de l'appareil orthodontique porté par le patient. Les indicateurs qui ne sont pas liés à cette vérification, par exemple l'indication des zones de carie, ne sont alors pas nécessaires.

Dans un mode de réalisation, avant l'étape d), l'opérateur saisit des informations complémentaires dans la base de données, en fonction de l'utilisation du procédé visée. Par exemple, il précise un emplacement pour attacher un article décoratif ou pour fraiser une dent. Les indicateurs correspondants peuvent avantageusement être ajoutés à la scène dentaire virtuelle.

Par exemple, une flèche peut pointer vers une zone d'une dent afin d'indiquer à l'opérateur l'endroit où il doit fixer un appareil orthodontique ou un article décoratif, ou sur lequel il doit agir, par exemple au moyen d'un outil comme une fraise.

Un indicateur peut également représenter, de manière réaliste ou non, une partie non visible d'un élément physique de la scène dentaire réelle (et qui, n'étant pas visible par l'opérateur, n'appartient donc pas à la scène dentaire réelle), par exemple une racine d'une dent, un os ou un nerf dentaire.

Les éléments qui constituent la scène dentaire virtuelle sont positionnés dans cette scène en fonction des éléments de la scène dentaire réelle, et donc en l'occurrence, en fonction de l'image de référence. En particulier, les représentations d'éléments physiques réels sont de préférence positionnées dans la scène dentaire virtuelle de manière qu'à l'étape e), ils se superposent, de manière précise, avec ces éléments physiques réels ou avec leurs représentations sur l'image actualisée.

De même, un indicateur apportant une information spécifique à un élément physique, par exemple spécifique à une dent, est de préférence positionné de manière qu'à l'étape e) il apparaisse à proximité immédiate ou superposé à cet élément physique, par exemple à cette dent.

Des indicateurs peuvent être disposés de manière à être affichés, à l'étape suivante, aux endroits où des différences ont été identifiées entre l'image de référence et l'image actualisée. Par exemple, on peut afficher une marque rouge aux emplacements où un appareil orthodontique s'est décollé des dents.

Dans un mode de réalisation, le contexte est évolutif et la nature et/ou l'emplacement, dans la scène dentaire virtuelle, des indicateurs sont adaptés en conséquence. En particulier, la nature et/ou l'emplacement des indicateurs peuvent être déterminés en fonction de la nature et/ou de l'emplacement d'indicateurs affichés dans des scènes dentaires virtuelles antérieures, c'est-à-dire déterminées lors de cycles c)-e) (ou A) à C)) précédents, et/ou en fonction de l'intervalle temporel avec lesdites scènes dentaires virtuelles antérieures, et/ou en fonction de différences entre les images actualisées de cycles c)-e) (ou A) à C)) successifs.

Par exemple, le procédé peut être utilisé pour guider une action de l'opérateur avec un outil, par exemple un perçage au moyen d'un foret. Un élément de la scène dentaire virtuelle peut être une flèche indiquant l'orientation du foret et la position de son extrémité libre. La base de données peut contenir un protocole fixant par exemple le point de pénétration dans la dent, la trajectoire et la vitesse optimale d'avancement du foret. A chaque cycle des étapes c) à e) (ou A) à C)), l'emplacement de l'élément dans la scène dentaire virtuelle est alors déterminé en fonction de son emplacement dans les scènes dentaires virtuelles déterminées antérieurement et, de préférence, en fonction de l'intervalle temporel entre ladite scène dentaire virtuelle et lesdites scènes dentaires virtuelles antérieures.

De préférence encore, l'outil, par exemple le foret, qui apparaît sur les images actualisées successives, est identifié sur ces images et l'emplacement de l'élément dans la scène dentaire virtuelle est adapté pour tenir compte de la position réelle de l'outil. Avantageusement, la représentation de l'indicateur dans la scène dentaire virtuelle peut donc évoluer en fonction de la position réelle de l'outil. Notamment, elle peut ainsi s'adapter à la vitesse de progression réelle ou à la trajectoire réelle de l'outil.

Dans un mode de réalisation, l'apparence d'un élément de la scène dentaire virtuelle évolue en fonction du contexte.

Par exemple, on identifie un élément de la scène dentaire virtuelle qui ne respecte pas un critère d'évaluation, par exemple parce qu'il dépasse un seuil de risque, puis on attire l'attention vers cet élément dans la scène dentaire virtuelle. Par exemple, la couleur de l'élément peut être modifiée si l'analyse du contexte montre que la situation devient dangereuse, par exemple parce que l'outil ne suit pas la trajectoire souhaitée ou parce que l'appareil orthodontique conduirait à une correction excessive du positionnement des dents.

Dans un mode de réalisation, la scène dentaire virtuelle ne comporte pas l'image de référence. L'image de référence peut être utilisée pour déterminer la nature et/ou l'emplacement des éléments dans la scène dentaire virtuelle. Mais elle n'est pas présentée à l'observateur à l'étape e) (ou C)).

Dans un mode de réalisation, la scène dentaire virtuelle est déterminée en fonction d'un instant de simulation passé ou futur. En particulier, elle peut comporter une représentation, de préférence réaliste, d'un ou plusieurs éléments de la scène dentaire réelle correspondante dans une configuration passée ou future.

Dans un mode de réalisation, elle comporte une représentation de l'arcade et/ou une représentation d'un appareil orthodontique porté par les dents de l'arcade telle que mesurée ou simulée à un instant de simulation passé.

Dans un mode de réalisation, elle comporte une représentation de l'arcade et/ou une représentation d'un appareil orthodontique porté par les dents de l'arcade telle qu'anticipée pour un instant de simulation futur.

La simulation à un instant de simulation passé ou futur peut être réalisée par tout logiciel de simulation ou par un opérateur, par exemple par un professionnel des soins dentaires.

La scène dentaire virtuelle peut par exemple représenter les contours des dents, le positionnement d'un appareil orthodontique ou la couleur des dents à l'instant de simulation. La présentation de la scène dentaire virtuelle sur la scène dentaire réelle, de préférence en transparence sur la scène dentaire réelle, permet ainsi de facilement détecter l'évolution entre l'instant actualisé et l'instant de simulation.

Lorsqu'un indicateur représente une partie non visible d'un élément physique de la scène dentaire réelle, par exemple une racine d'une dent, l'information relative à cet indicateur est de préférence incluse dans le modèle de référence. Par exemple, le modèle de référence peut être un modèle de l'arcade dentaire dans lequel les couronnes des dents, mais aussi les racines des dents sont modélisées, par exemple au moyen d'un scanner à faisceau conique (*« Cone beam »*)*.* Le dispositif selon l'invention permet ainsi à l'opérateur de visualiser des parties de l'arcade dentaire du patient qui ne sont pas visibles sur la scène dentaire réelle. Avantageusement, ces parties non visibles sont représentées dans leur position relative réelle par rapport aux éléments physiques de la scène dentaire réelle. Par exemple, l'opérateur voit les racines et les nerfs des dents qu'il observe dans la bouche du patient. Il peut ainsi éviter une interaction avec un outil qu'il manipule ou adapter un appareil orthodontique pour limiter le risque d'une collision entre les racines de dents adjacentes, et donc limiter le risque de rhizalyse. Par exemple, lors d'une injection au moyen d'une seringue, l'opérateur peut introduire précisément l'aiguille, jusqu'à atteindre une racine dentaire, sans toucher un nerf dentaire. Les douleurs ressenties par le patient en sont limitées.

**A l'étape e),** on présente, en fonction de l'image de référence, la scène dentaire virtuelle en superposition sur la scène dentaire réelle ou sur la représentation de la scène dentaire réelle dans l'image actualisée.

La scène dentaire virtuelle est affichée sur un écran.

Lorsque l'écran est transparent, en particulier dans le mode de réalisation dans lequel il est intégré dans des lunettes, l'opérateur peut ainsi observer la scène dentaire virtuelle sur l'écran et la scène dentaire réelle à travers l'écran, en transparence derrière la scène dentaire virtuelle.

Lorsque l'écran est opaque, en particulier dans le mode de réalisation dans lequel l'écran est l'écran d'un téléphone portable, d'un ordinateur portable, d'un casque de réalité virtuelle ou d'une tablette, l'opérateur peut ainsi simultanément observer sur l'écran l'image actualisée et la scène dentaire virtuelle.

La scène dentaire virtuelle peut être suffisamment transparente pour laisser apparaître la scène dentaire réelle ou l'image actualisée sous elle.

Alternativement, la scène dentaire virtuelle peut être opaque, de manière à ne pas laisser apparaître la scène dentaire réelle ou l'image actualisée qui s'étend derrière ou sous elle. L'opacité de la scène dentaire virtuelle est particulièrement avantageuse pour simuler une situation dentaire passée ou future, par exemple pour visualiser le blanchiment des dents sous l'effet d'un traitement à cet effet, ou le déplacement d'une dent, par exemple sous l'effet d'un traitement orthodontique ou en l'absence d'un traitement orthodontique, par exemple pour visualiser un déchaussement d'une dent. Lorsque la scène dentaire virtuelle est présentée sur l'image actualisée, elle peut être ajoutée au-dessus de l'image actualisée ou, de manière équivalente, l'image actualisée peut être remplacée par une image retraitée informatiquement pour faire apparaître la scène dentaire virtuelle.

Par « présentation de la scène dentaire virtuelle en superposition sur la représentation de la scène dentaire réelle dans l'image actualisée », il faut donc inclure toute présentation faisant apparaître la scène dentaire virtuelle sur la représentation de la scène dentaire réelle, y compris l'affichage d'une image retraitée.

Lorsque l'écran est la glace d'un miroir, l'opérateur peut simultanément observer sur l'écran son reflet et la scène dentaire virtuelle, superposée à son reflet. En particulier, le patient peut visualiser l'effet d'un traitement dentaire, thérapeutique ou non.

La scène dentaire virtuelle est présentée en fonction de l'image de référence et donc, indirectement, en fonction de l'image actualisée. En particulier, elle est positionnée, ou « cadrée », par rapport à la scène dentaire réelle en fonction de l'image de référence.

La scène dentaire virtuelle est présentée en superposition, ou « en registre », avec la scène dentaire réelle ou avec sa représentation sur l'image actualisée, ce qui facilite la comparaison. Autrement dit, les éléments de la scène dentaire virtuelle qui correspondent à des éléments de la scène dentaire réelle sont sensiblement exactement superposés à ces derniers ou aux représentations de ces derniers. Notamment lorsque la scène dentaire virtuelle est présentée en transparence, les différences entre la scène dentaire réelle ou sa représentation d'une part et la scène dentaire virtuelle d'autre part apparaissent ainsi clairement à l'opérateur. Les indicateurs lui présentent par ailleurs des informations facilitant l'analyse de la situation.

De préférence, pour présenter la scène dentaire virtuelle en registre sur la scène dentaire réelle ou sur sa représentation sur l'image actualisée, on identifie sur l'image actualisée des marques caractéristiques dont la position par rapport à la scène dentaire virtuelle est connue. Par exemple, on identifie des extrémités de dent ou des formes caractéristiques de dents représentées à la fois sur l'image actualisée et sur la scène dentaire virtuelle. L'image de référence à partir de laquelle la scène dentaire virtuelle a été déterminée peut en particulier être utilisée pour identifier la position des marques caractéristiques dans la scène virtuelle. A l'étape e) (ou C)), on superpose ensuite les représentations de ces marques caractéristiques sur l'image actualisée et dans la scène dentaire virtuelle, ce qui permet de positionner précisément la scène dentaire virtuelle par rapport aux marques caractéristiques représentées sur l'image actualisée.

De préférence, le procédé reprend ensuite à l'étape c) (ou A)) moins de 3 secondes, de préférence moins de 1 s, moins de 0,5 s, moins de 0,2 s, de préférence moins de 0,1 s après l'étape e) (ou C)). De préférence, le cycle des étapes c) à e) (ou A) à C)) est effectué en temps réel, sans interruption.

### Exemples d'applications

### Assistance en temps réel lors d'une intervention sur les dents

Notamment lorsque l'appareil d'acquisition d'images est sous la forme de lunettes, l'invention peut être utilisée lors d'une intervention sur les dents, par le professionnel des soins dentaires portant les lunettes.

Dans un mode de réalisation, le procédé est utilisé dans le cadre d'une opération d'abrasion d'une dent, ou « stripping », ou de fraisage. De préférence, la scène dentaire virtuelle représente la dent dans sa forme finale, après l'abrasion ou le fraisage à effectuer. De préférence, le professionnel des soins dentaires aperçoit le contour de la dent dans sa forme finale en superposition précise, c'est-à-dire en registre, sur la dent réelle. Cette information lui permet d'usiner la dent avec une parfaite précision.

Dans un mode de réalisation, le procédé est utilisé pour fixer une attache dentaire, ou « bracket », sur une dent. De préférence, la scène dentaire virtuelle représente l'attache dans sa position finale, après sa fixation sur la dent. De préférence, le professionnel des soins dentaires aperçoit le contour virtuel de l'attache dans sa position finale par rapport à la dent réelle. Cette information lui permet de placer l'attache réelle en superposition exacte avec l'attache de la scène dentaire virtuelle, et donc de la positionner sur la dent réelle avec une parfaite précision.

Le procédé peut être également utilisé pour fixer un article décoratif sur les dents.

Lors de l'intervention, l'invention permet à l'opérateur, en particulier un professionnel des soins dentaires, d'accéder à des informations pertinentes. Ces informations lui sont présentées, de préférence en temps réel, en superposition avec la scène dentaire réelle qu'il observe directement ou sur l'image actualisée. L'invention permet ainsi d'accroitre considérablement la qualité de l'analyse de la situation, et, le cas échéant, d'adapter une intervention en conséquence.

### Amélioration de l'observance et utilisation à des fins pédagogiques

Dans un mode de réalisation, le procédé est utilisé pour obtenir une meilleure acceptation du traitement par le patient. En particulier, il peut être utilisé pour simuler, de manière réaliste, un traitement dentaire. De préférence, l'appareil d'acquisition d'images est alors un téléphone portable, un ordinateur portable, une tablette ou un miroir.

Avec un téléphone portable, un ordinateur portable ou une tablette, le patient se filme les arcades dentaires, comme lorsqu'il prend un selfie. Avec un miroir, le patient est de préférence filmé à travers la surface du miroir, ce qui permet à l'image actualisée de représenter fidèlement le reflet R observé par le patient.

En superposition avec les images actualisées qui se succèdent sur l'écran du téléphone, de l'ordinateur ou de la tablette, ou en superposition avec son reflet sur le miroir, le patient aperçoit des scènes dentaires virtuelles correspondantes, qui représentent une étape du traitement dentaire à un instant de simulation. Par exemple, l'instant de simulation peut être l'instant marquant le début du traitement et la scène dentaire virtuelle peut représenter un appareil orthodontique que le patient envisage de porter. Le patient peut alors visualiser son visage après que l'appareil orthodontique aura été mis en position dans sa bouche. En tournant la tête ou en déplaçant le téléphone portable, l'ordinateur portable ou la tablette, ou en se déplaçant devant le miroir il peut facilement modifier la direction d'observation, la scène dentaire virtuelle s'adaptant en temps réel.

De préférence, le patient peut modifier l'instant de simulation par interaction avec l'écran, par exemple en déplaçant un curseur virtuel. La scène dentaire virtuelle est adaptée en conséquence, notamment pour tenir compte de l'effet du traitement. En agissant sur le curseur, le patient peut donc simuler le déroulement du traitement, c'est-à-dire visualiser par exemple comment les dents vont se déplacer et/ou changer de couleur, notamment en cas de traitement de blanchiment des dents ou si les dents sont soumises à un agent colorant, par exemple si le patient fume ou boit de grandes quantités de thé ou de café. Le déplacement du curseur lui permet ainsi de simuler une accélération du temps.

Cette simulation facilite la prise de décision par le patient et constitue avantageusement une incitation à suivre le traitement.

Dans un mode de réalisation, le patient peut modifier la valeur d'autres paramètres de la simulation. Par exemple, il peut modifier des valeurs de paramètres du traitement.

Par exemple, il peut simuler
- l'effet d'un ou plusieurs appareils orthodontiques, notamment afin de choisir celui qui lui convient le mieux ;
- l'effet d'un arrêt, temporaire ou définitif, d'un traitement en cours ;
- l'effet du respect plus ou moins strict des consignes reçues, notamment pour visualiser l'effet d'une modification de la fréquence et/ou de la durée et/ou de la technique de brossage des dents, ou l'effet d'un retard dans un changement de gouttière orthodontique et/ou d'un retard pour prendre rendez-vous chez le professionnel des soins dentaires.

Il peut ainsi simuler l'effet d'une mauvaise observance de la prescription médicale, ce qui est facteur d'amélioration de l'observance.

Dans un mode de réalisation, la scène dentaire virtuelle fait apparaître des avertissements, de préférence sous la forme d'un message ou d'une mise en évidence d'un élément de la scène dentaire virtuelle, par exemple en faisant apparaitre en rouge une zone qui doit être brossée plus soigneusement.

De manière générale, l'invention peut être utilisée pour évaluer la situation dentaire du patient, notamment pour contrôler le déroulement d'un traitement dentaire, et/ou, hors du cadre d'un traitement dentaire, pour contrôler une évolution de la couleur et/ou de la forme et/ou de l'agencement des dents ou des gencives.

### Analyse d'une situation dentaire évolutive

Dans un mode de réalisation, le procédé est utilisé pour visualiser l'abrasion subie par une dent, par exemple du fait d'un bruxisme. De préférence, la scène dentaire virtuelle représente la dent dans sa forme antérieure, avant l'abrasion. De préférence, l'opérateur, généralement le patient ou le professionnel des soins dentaires, aperçoit le contour de la dent dans sa forme antérieure en superposition sur la dent réelle ou sur sa représentation sur l'image actualisée. Cette information lui permet de visualiser immédiatement et précisément l'ampleur de cette usure, notamment lorsque la scène dentaire virtuelle est présentée en transparence.

Dans un mode de réalisation, le procédé est utilisé pour visualiser une déformation de la gencive, et en particulier une récession de la gencive. De préférence, la scène dentaire virtuelle représente la gencive dans sa forme initiale, avant sa déformation. Ainsi, l'opérateur, généralement le patient ou le professionnel des soins dentaires, aperçoit le contour de la gencive dans sa forme initiale en superposition sur la gencive réelle ou sur sa représentation. Cette information lui permet de visualiser immédiatement et précisément cette déformation, notamment lorsque la scène dentaire virtuelle est présentée en transparence.

Bien entendu, l'invention n'est pas limitée aux modes de réalisation décrits ci-dessus et représentés.

En particulier, le patient n'est pas limité à un être humain. Un dispositif selon l'invention peut être utilisé pour un autre animal.

L'invention n'est pas limitée à un dispositif mettant en œuvre un procédé comportant des étapes a) à e). En particulier, la recherche d'une image de référence n'est pas indispensable à la mise en œuvre de l'invention. Si une image de référence n'est pas disponible, les éléments qui constituent la scène dentaire virtuelle peuvent être positionnés sur la scène dentaire réelle ou sa représentation, en fonction de l'image actualisée.

Une scène dentaire virtuelle peut être déterminée en fonction de plusieurs images de référence obtenues à partir de plusieurs images actualisées respectives acquises simultanément ou à des instants actualisés différents, par exemple successifs.

## Revendications

1. Dispositif pour la mise en œuvre d'un procédé d'analyse d'une situation dentaire réelle d'un patient, à des fins de simulation, le procédé comportant une série de cycles d'étapes A) à C), un cycle d'étapes A) à C) comportant les étapes successives suivantes :
A) à un instant actualisé, acquisition d'une image actualisée représentant une scène dentaire réelle telle qu'observée par un opérateur ;
B) détermination d'une scène dentaire virtuelle en fonction de la représentation de la scène dentaire réelle sur l'image actualisée, la scène dentaire virtuelle comportant une représentation de l'arcade du patient et/ou une représentation d'un appareil orthodontique porté par les dents de l'arcade telle qu'anticipée pour un instant de simulation futur, l'instant de simulation étant postérieur à l'instant actualisé de plus de 1 jour ;
C) présentation, sur un écran, de la scène dentaire virtuelle en transparence et en superposition sur la scène dentaire réelle, ou
affichage de l'image actualisée sur un écran et présentation de la scène dentaire virtuelle en superposition avec la représentation de la scène dentaire réelle sur l'image actualisée affichée sur l'écran, en transparence ou non sur ladite représentation ;
une étape A) étant reprise moins de 5 secondes après l'étape C),
le dispositif comportant :
- une caméra (16) configurée pour mettre en œuvre l'étape A) ;
- une unité de traitement (12) configurée pour mettre en œuvre l'étape B) ;
- un écran (18) pour mettre en œuvre l'étape C) ;
la caméra (16), l'unité de traitement (12) et l'écran (18) étant configurés pour communiquer entre eux pour mettre en œuvre lesdites étapes.

2. Dispositif selon la revendication précédente, dans lequel l'écran (18)
- est un écran tactile et l'intervalle temporel entre l'instant actualisé et l'instant de simulation est modifiable par interaction avec l'écran, de préférence par glissement d'un doigt sur ledit écran, et/ou
- comporte un champ de saisie de l'instant de simulation.

3. Dispositif selon l'une quelconque des revendications précédentes, dans lequel, entre deux cycles d'étapes A) à C), l'intervalle temporel entre l'instant actualisé et l'instant de simulation est modifié.

4. Dispositif selon l'une quelconque des revendications précédentes, dans lequel la caméra et/ou l'écran sont intégrés dans un téléphone portable, un ordinateur portable, un casque de réalité virtuelle, une tablette, un appareil photo ou un miroir (17), la glace (19) du miroir, de préférence sans tain, constituant ledit écran, ou dans lequel l'écran et la caméra sont indépendants l'un de l'autre, c'est-à-dire ne sont pas rigidement solidaire l'un de l'autre.

5. Dispositif selon l'une quelconque des revendications précédentes, dans lequel, à l'étape B), pour l'instant de simulation, une couleur et/ou une forme et/ou une dimension et/ou une position d'une partie visible ou invisible de la bouche, de préférence d'une mâchoire, d'une couronne dentaire et/ou d'une racine dentaire et/ou de la gencive du patient, est/sont déterminée(s), et la scène dentaire virtuelle est déterminée de manière qu'elle reproduise ladite couleur et/ou forme et/ou position.

6. Dispositif selon l'une quelconque des revendications précédentes, dans lequel, à l'étape B), un élément de la scène dentaire virtuelle qui ne respecte pas un critère d'évaluation est identifié, et cet élément dans la scène dentaire virtuelle est mis en évidence et/ou un message relatif audit élément, de préférence une consigne à suivre par le patient, est ajouté à la scène dentaire virtuelle.

7. Dispositif selon la revendication immédiatement précédente, dans lequel le critère d'évaluation est relatif à l'état de santé du patient et/ou au positionnement d'un appareil orthodontique porté par le patient et/ou à l'état d'un appareil orthodontique porté par le patient et/ou à un coût pour un traitement dentaire.

8. Dispositif selon l'une quelconque des revendications précédentes, dans lequel, à l'étape B), la scène dentaire virtuelle est déterminée en fonction d'une valeur d'au moins un paramètre de traitement modifiable par un opérateur.

9. Dispositif selon la revendication immédiatement précédente, dans lequel le paramètre de traitement est relatif à un port d'un appareil orthodontique par le patient et/ou au respect d'une consigne de traitement par le patient.

10. Dispositif selon l'une quelconque des revendications précédentes, pour la mise en œuvre des étapes successives suivantes :
a) à un instant de référence, génération d'un modèle de référence modélisant numériquement au moins une arcade du patient;
b) modification du modèle de référence ;
c) à l'instant actualisé, acquisition de ladite image actualisée, suivant l'étape A) ;
d) recherche, par observation du modèle de référence modifié, d'une image de référence présentant une concordance maximale avec l'image actualisée, puis détermination, en fonction de l'image de référence, de la scène dentaire virtuelle, suivant l'étape B);
e) présentation de la scène dentaire virtuelle suivant l'étape C) le dispositif comportant des moyens pour générer et modifier un modèle de référence suivant les étapes a) et b), ces moyens comportant de préférence un scanner et un ordinateur,
la caméra étant configurée pour mettre en œuvre l'étape c), l'unité de traitement pour mettre en œuvre l'étape d) et l'écran pour mettre en œuvre l'étape e).

11. Dispositif selon la revendication immédiatement précédente, l'unité de traitement étant configurée pour
- rechercher, pour chaque image actualisée reçue de la caméra, par observation du modèle de référence modifié, une image de référence présentant une concordance maximale avec ladite image actualisée ;
- déterminer, en fonction de l'image de référence, une scène dentaire virtuelle ; et
- transmettre ladite scène dentaire virtuelle aux moyens de présentation,
l'écran étant configuré pour faire apparaitre la scène dentaire virtuelle en superposition sur la scène dentaire réelle ou sur la représentation de la scène dentaire réelle dans l'image actualisée.

12. Dispositif selon l'une quelconque des deux revendications immédiatement précédentes, dans lequel, à l'étape a), le modèle de référence est généré en scannant une arcade du patient ou un modèle physique de ladite arcade ou en choisissant un modèle générique d'arcade dans une base de modèles génériques, puis en déformant ledit modèle générique en fonction de mesures ou d'observations faites sur le patient.

13. Dispositif selon l'une quelconque des trois revendications immédiatement précédentes, dans lequel, à l'étape b), le modèle de référence est modifié pour simuler l'effet de l'écoulement du temps entre l'instant de référence et l'instant de simulation.

14. Dispositif selon l'une quelconque des revendications précédentes, dans lequel, la simulation est obtenue au moyen d'un ordinateur, au moyen d'un algorithme d'intelligence artificielle.

15. Dispositif selon l'une quelconque des revendications précédentes configuré pour simuler :
- l'effet d'un ou plusieurs appareils orthodontiques ;
- l'effet d'un arrêt, temporaire ou définitif, d'un traitement, thérapeutique ou non, en cours ;
- l'effet d'un traitement futur, thérapeutique ou non, de préférence pour modifier la couleur ou la position des dents ;
- l'effet d'une modification de la fréquence et/ou de la durée et/ou de la technique de brossage des dents, ou l'effet d'un retard dans un changement de gouttière orthodontique,
- l'effet d'un traitement de blanchiment des dents ou l'effet de la cigarette.

## Patentansprüche

1. Vorrichtung zur Umsetzung eines Verfahrens zur Analyse einer realen Zahnsituation eines Patienten zu Simulationszwecken, wobei das Verfahren eine Reihe von Zyklen aus Schritten A) bis C) umfasst, wobei ein Zyklus aus Schritten A) bis C) die folgenden aufeinanderfolgenden Schritte umfasst:
A) zu einem aktualisierten Zeitpunkt, Erfassen eines aktualisierten Bildes, das eine reale Zahnszene darstellt, wie sie von einem Bediener betrachtet wird;
B) Bestimmen einer virtuellen Zahnszene in Abhängigkeit von der Darstellung der realen Zahnszene auf dem aktualisierten Bild, wobei die virtuelle Zahnszene eine Darstellung des Zahnbogens des Patienten umfasst und/oder eine Darstellung eines von den Zähnen des Zahnbogens getragenen orthodontischen Geräts, wie für einen zukünftigen Simulationszeitpunkt antizipiert, wobei der Simulationszeitpunkt um mehr als 1 Tag nach dem aktualisierten Zeitpunkt liegt;
C) Präsentieren, auf einem Bildschirm, der virtuellen Zahnszene in Transparenz und in Überlagerung über der realen Zahnszene oder
Anzeigen des aktualisierten Bildes auf einem Bildschirm und Präsentieren der virtuellen Zahnszene in Überlagerung mit der Darstellung der realen Zahnszene auf dem aktualisierten Bild, das auf dem Bildschirm, in Transparenz oder nicht über der Darstellung, angezeigt wird;
wobei ein Schritt A) weniger als 5 Sekunden nach Schritt C) wiederaufgegriffen wird, wobei die Vorrichtung umfasst:
- eine Kamera (16), die dazu ausgestaltet ist, Schritt A) umzusetzen;
- eine Verarbeitungseinheit (12), die dazu ausgestaltet ist, Schritt B) umzusetzen;
- einen Bildschirm (18), der dazu ausgestaltet ist, Schritt C) umzusetzen;
wobei die Kamera (16), die Verarbeitungseinheit (12) und der Bildschirm (18) dazu ausgestaltet sind, untereinander zu kommunizieren, um die Schritte umzusetzen.

2. Vorrichtung nach dem vorhergehenden Anspruch, bei welcher der Bildschirm (18)
- ein Touchscreen ist und das Zeitintervall zwischen dem aktualisierten Zeitpunkt und dem Simulationszeitpunkt durch Interaktion mit dem Bildschirm änderbar ist, bevorzugt durch Gleiten eines Fingers auf dem Bildschirm, und/oder
- ein Eingabefeld für den Simulationszeitpunkt umfasst.

3. Vorrichtung nach einem der vorhergehenden Ansprüche, bei der zwischen zwei Zyklen aus Schritten A) bis C) das Zeitintervall zwischen dem aktualisierten Zeitpunkt und dem Simulationszeitpunkt geändert wird.

4. Vorrichtung nach einem der vorhergehenden Ansprüche, bei der die Kamera und/oder der Bildschirm in ein Mobiltelefon, einen Laptop, eine Virtual-Reality-Brille, ein Tablet, einen Fotoapparat oder einen Spiegel (17) integriert sind, wobei die Scheibe (19) des Spiegels, bevorzugt des Einwegspiegels, den Bildschirm bildet, oder bei welcher der Bildschirm und die Kamera unabhängig voneinander sind, d. h. nicht starr miteinander verbunden sind.

5. Vorrichtung nach einem der vorhergehenden Ansprüche, bei der beim Schritt B) für den Simulationszeitpunkt eine Farbe und/oder eine Form und/oder ein Maß und/oder eine Position eines sichtbaren oder unsichtbaren Teils des Mundes, bevorzugt eines Kiefers, einer Zahnkrone und/oder einer Zahnwurzel und/oder des Zahnfleisches des Patienten, bestimmt wird/werden und die virtuelle Zahnszene so bestimmt wird, dass sie die Farbe und/oder Form und/oder Position wiedergibt.

6. Vorrichtung nach einem der vorhergehenden Ansprüche, bei der beim Schritt B) ein Element der virtuellen Zahnszene, das ein Bewertungskriterium nicht erfüllt, ermittelt wird und dieses Element in der virtuellen Zahnszene hervorgehoben wird, und/oder eine Meldung bezüglich des Elements, bevorzugt eine vom Patienten zu befolgende Anweisung, zu der virtuellen Zahnszene hinzugefügt wird.

7. Vorrichtung nach dem unmittelbar vorhergehenden Anspruch, bei der sich das Bewertungskriterium auf den Gesundheitszustand des Patienten und/oder auf die Positionierung eines von dem Patienten getragenen orthodontischen Geräts und/oder auf den Zustand eines von dem Patienten getragenen orthodontischen Geräts und/oder auf die Kosten für eine Zahnbehandlung bezieht.

8. Vorrichtung nach einem der vorhergehenden Ansprüche, bei der beim Schritt B) die virtuelle Zahnszene in Abhängigkeit von einem Wert mindestens eines Behandlungsparameters bestimmt wird, der von einem Bediener änderbar ist.

9. Vorrichtung nach dem unmittelbar vorhergehenden Anspruch, bei welcher sich der Behandlungsparameter auf ein Tragen eines orthodontischen Geräts durch den Patienten und/oder auf die Beachtung einer Behandlungsanweisung durch den Patienten bezieht.

10. Vorrichtung nach einem der vorhergehenden Ansprüche zur Umsetzung der folgenden aufeinanderfolgenden Schritte:
a) zu einem Referenzzeitpunkt, Erzeugen eines Referenzmodells, das mindestens einen Zahnbogen des Patienten digital abbildet;
b) Ändern des Referenzmodells;
c) zu dem aktualisierten Zeitpunkt, Erfassen des aktualisierten Bildes gemäß Schritt A);
d) Suchen, durch Betrachten des geänderten Referenzmodells, eines Referenzbildes, das eine maximale Übereinstimmung mit dem aktualisierten Bild aufweist, dann Bestimmen, in Abhängigkeit von dem Referenzbild, der virtuellen Zahnszene gemäß Schritt B) ;
e) Präsentation der virtuellen Zahnszene gemäß Schritt C)
wobei die Vorrichtung Mittel umfasst, um ein Referenzmodell gemäß den Schritten a) und b) zu erzeugen und zu ändern, wobei diese Mittel bevorzugt einen Scanner und einen Computer umfassen,
wobei die Kamera dazu ausgestaltet ist, den Schritt c) umzusetzen, die Verarbeitungseinheit dazu, den Schritt d) umzusetzen, und der Bildschirm dazu, den Schritt e) umzusetzen.

11. Vorrichtung nach dem unmittelbar vorhergehenden Anspruch, wobei die Verarbeitungseinheit dazu ausgestaltet ist,
- für jedes von der Kamera empfangene aktualisierte Bild durch Betrachten des geänderten Referenzmodells ein Referenzbild zu suchen, das eine maximale Übereinstimmung mit dem aktualisierten Bild aufweist;
- in Abhängigkeit von dem Referenzbild eine virtuelle Zahnszene zu bestimmen, und
- die virtuelle Zahnszene zu den Präsentationsmitteln zu übertragen,
wobei der Bildschirm dazu ausgestaltet ist, die virtuelle Zahnszene in Überlagerung über der realen Zahnszene oder über der Darstellung der realen Zahnszene in dem aktualisierten Bild erscheinen zu lassen.

12. Vorrichtung nach einem der beiden unmittelbar vorhergehenden Ansprüche, bei der im Schritt a) das Referenzmodell erzeugt wird, indem ein Zahnbogen des Patienten oder ein physisches Modell des Zahnbogens gescannt wird oder indem ein generisches Zahnbogenmodell aus einer Datenbank mit generischen Modellen gewählt wird und dann das generische Modell in Abhängigkeit von am Patienten vorgenommenen Messungen oder Betrachtungen verformt wird.

13. Vorrichtung nach einem der drei unmittelbar vorhergehenden Ansprüche, bei der im Schritt b) das Referenzmodell geändert wird, um den Effekt des Verstreichens der Zeit zwischen dem Referenzzeitpunkt und dem Simulationszeitpunkt zu simulieren.

14. Vorrichtung nach einem der vorhergehenden Ansprüche, bei der die Simulation mittels eines Computers, mittels eines Algorithmus der künstlichen Intelligenz erhalten wird.

15. Vorrichtung nach einem der vorhergehenden Ansprüche, die dazu ausgestaltet ist, zu simulieren:
- den Effekt eines oder mehrerer orthodontischer Geräte;
- den Effekt eines vorübergehenden oder endgültigen Aussetzens einer laufenden therapeutischen oder nicht therapeutischen Behandlung;
- den Effekt einer zukünftigen therapeutischen oder nicht therapeutischen Behandlung, bevorzugt, um die Farbe oder die Stellung der Zähne zu ändern;
- den Effekt einer Änderung der Häufigkeit und/oder der Dauer und/oder der Technik des Zähneputzens oder den Effekt einer Verzögerung beim Wechseln einer orthodontischen Schiene,
- den Effekt einer Zahnbleichbehandlung oder den Effekt der Zigarette.

## Claims

1. Device for implementing a method for analysing an actual dental situation of a patient, for simulation purposes, the method comprising a series of cycles of steps A) to C), a cycle of steps A) to C) comprising the following successive steps:
A) at an update time, acquiring an update image representing an actual dental scene such as observed by an operator;
B) determining a virtual dental scene depending on the representation of the actual dental scene in the update image, the virtual dental scene comprising a representation of the arch of the patient and/or a representation of an orthodontic appliance borne by the teeth of the arch such as anticipated for a future simulation time, the simulation time being after the update time by more than one day;
C) presenting, on a screen, the virtual dental scene in see-through and in superposition on the actual dental scene, or
displaying the update image on a screen and presenting the virtual dental scene in superposition with the representation of the actual dental scene in the update image displayed on the screen, in see-through or not on said representation;
a step A) being repeated less than 5 seconds after step C),
the device comprising:
- a camera (16) configured to implement step A);
- a processing unit (12) configured to implement step B) ;
- a screen (18) for implementing step C);
the camera (16), the processing unit (12) and the screen (18) being configured to communicate with one another to implement said steps.

2. Device according to the preceding claim, wherein the screen (18)
- is a touchscreen and the time interval between the update time and the simulation time is modifiable via interaction with the screen, and preferably by sliding a finger over said screen, and/or
- comprises a field for inputting the simulation time.

3. Device according to any one of the preceding claims, wherein, between two cycles of steps A) to C), the time interval between the update time and the simulation time is modified.

4. Device according to any one of the preceding claims, wherein the camera and/or the screen are integrated into a mobile telephone, a laptop, a virtual reality headset, a tablet, a camera or a mirror (17), the mirror glass (19) of the mirror, which is preferably a one-way mirror, forming said screen, or wherein the screen and the camera are independent of each other, i.e. not rigidly secured to each other.

5. Device according to any one of the preceding claims, wherein, in step B), for the simulation time, a colour and/or a shape and/or a dimension and/or a position of a visible or invisible portion of the mouth, preferably of a jaw, of a crown and/or of a root and/or of the gum of the patient, is/are determined, and the virtual dental scene is determined so that it reproduces said colour and/or shape and/or position.

6. Device according to any one of the preceding claims, wherein, in step B), an element of the virtual dental scene that does not meet an evaluation criterion is identified, and this element in the virtual dental scene is highlighted and/or a message relating to said element, preferably an instruction to be followed by the patient, is added to the virtual dental scene.

7. Device according to the immediately preceding claim, wherein the evaluation criterion relates to the state of health of the patient and/or to the positioning of an orthodontic appliance worn by the patient and/or to the state of an orthodontic appliance worn by the patient and/or to a cost of a dental treatment.

8. Device according to any one of the preceding claims, wherein, in step B), the virtual dental scene is determined depending on a value of at least one treatment parameter that is modifiable by an operator.

9. Device according to the immediately preceding claim, wherein the treatment parameter relates to whether an orthodontic appliance is worn by the patient and/or to whether a treatment instruction is followed by the patient.

10. Device according to any one of the preceding claims, for implementing the following successive steps:
a) at a reference time, generating a reference model digitally modelling at least one arch of the patient;
b) modifying the reference model;
c) at the update time, acquiring said update image, according to step A);
d) seeking, via observation of the modified reference model, a reference image that best fits the update image, then determining, depending on the reference image, the virtual dental scene, according to step B) ;
e) presenting the virtual dental scene according to step C),
the device comprising means for generating and modifying a reference model according to steps a) and b), these means preferably comprising a scanner and a computer, the camera being configured to implement step c), the processing unit to implement step d) and the screen to implement step e).

11. Device according to the immediately preceding claim, the processing unit being configured to
- seek, for each update image received from the camera, via observation of the modified reference model, a reference image that best fits said update image;
- determine, depending on the reference image, a virtual dental scene; and
- transmit said virtual dental scene to the presenting means,
the screen being configured to make the virtual dental scene appear in superposition on the actual dental scene or on the representation of the actual dental scene in the update image.

12. Device according to either one of the two immediately preceding claims, wherein, in step a), the reference model is generated by scanning an arch of the patient or a physical model of said arch, or by choosing a generic arch model from a database of generic models, then by deforming said generic model depending on measurements or observations of the patient.

13. Device according to any one of the three immediately preceding claims, wherein, in step b), the reference model is modified to simulate the effect of the passage of time between the reference time and the simulation time.

14. Device according to any one of the preceding claims, wherein the simulation is obtained by means of a computer, or by means of an artificial-intelligence algorithm.

15. Device according to any one of the preceding claims, configured to simulate:
- the effect of one or more orthodontic appliances;
- the effect of temporary or permanent stoppage of a therapeutic or non-therapeutic treatment in progress;
- the effect of a future therapeutic or non-therapeutic treatment, preferably for modifying the colour or the position of the teeth;
- the effect of modifying the frequency of brushing of and/or the duration of brushing of and/or the technique used to brush the teeth, or the effect of a delay in a change of orthodontic retainer;
- the effect of a whitening treatment on the teeth or the effect of smoking.
